# EUROPEAN PATENT SPECIFICATION

(11) **EP 1 682 149 B1**
(45) Date of publication and mention of the grant of the patent: **02.12.2009**
(21) Application number: 04795782.4
(22) Date of filing: 21.10.2004
(51) Int. Cl.: A61K 31/52, A61K 9/08

(54) **INJECTABLE GABAPENTIN COMPOSITIONS**
GABAPENTIN-ZUSAMMENSETZUNGEN ZUR INJEKTION
COMPOSITIONS DE GABAPENTINE INJECTABLES

(30) Priority: 23.10.2003 US 513682 P; 23.10.2003 US 513681 P; 24.03.2004 US 808129
(43) Date of publication of application: 26.07.2006
(73) Proprietor: Medtronic, Inc., Minneapolis, MN 55432-5604 (US)
(72) Inventor: ELSBERRY, Dennis, D., Plymouth, Minnesota 55442 (US); CLARAHAN, David, A., Blaine, Minnesota 55449 (US); PAGE, Linda, M., Woodbury, Minnesota 55125 (US); LANE, Deanna, S., Columbia Heights, Minnesota 55421 (US); HILDEBRAND, Keith, R., Houlton, Wisconsin 54082 (US); RATNAYAKE, Jayantha, H., Blaine, Minnesota 55449 (US)
(74) Representative: Golding, Louise Ann
(86) International application number: PCT/US2004/034668
(87) International publication number: WO 2005/041976

(56) References cited:
- WO-A-02/100347
- WO-A-03/061656
- WO-A-03/070237
- US-A- 5 543 316

## Description

### RELATED APPLICATIONS

This application claims priority to Provisional Application Serial No. 60/513682, entitled "INJECTABLE GABAPENTIN COMPOSITIONS", filed October 23,2003, Provisional Application Serial No. 60/513681, entitled "INTRATHECAL GABAPENTIN FOR TREATMENT OF PAIN AND EPILEPSY", filed on October 23, 2003, and US Patent Application Serial No. 10/808,129, entitled "INJECTABLE GABAPENTIN COMPOSITIONS", filed on March 24, 2004.

### FIELD OF THE INVENTION

This application relates to injectable compositions and kits comprising gabapentin and to processes for producing the same.

### BACKGROUND OF THE INVENTION

Gabapentin is a pharmacological agent that mimics the effects of GABA (γ-aminobutyric acid), but gabapentin does not appear to bind a GABA receptor (e.g., GABA_{A} and GABA_{B} receptors) or have an effect on GABA uptake. Gabapentin has been found to interact with the alpha-2-delta (α₂δ) subunit of voltage-gated calcium channels. Many of the pharmacological effects of gabapentin may be due to its interaction with voltage-gated calcium channels. It is believed that gabapentin decreases calcium ion flow into a neuron, rendering the neuron less excitable. Inhibition of presynaptic calcium influx may prevent the release of neurotransmitters. Thus, like GABA, gabapentin can dampen overactive neural circuitry.

Solid formulations of gabapentin, such as NEURONTIN, are currently available for oral administration. Oral gabapentin has been primarily used to treat epilepsy although it has been used off-label to treat neuropathic pain and has recently received an FDA-approval for the treatment of one type of neuropathic pain, post-herpetic neuralgia. Some gabapentin can access the CNS when administered orally, because gabapentin is transported across the gut and the blood-brain barrier. It is believed that gabapentin is transported across the blood-brain barrier via an active and saturable L-amino acid transporter. Thus, the amount of gabapentin reaching CNS sites of action is limited. Because this transporter is saturable, even if the concentration of gabapentin in the plasma is increased, the amount which crosses the blood-brain barrier will remain constant

Solutions of gabapentin have been prepared for direct administration to the CNS in preclinical animal studies. In some studies, such solutions have been administered intrathecally as a single bolus or as multiple boluses. In these studies, the solutions contained gabapentin in varying concentrations, generally from about 1 mg/ml to about 30 mg/mL. One study (Wang and Yaksh, 1997) reported the use of a solution of 100 mg/ml gabapentin for a single 10 µl intrathecal bolus (1000 µg) injection in rats. Wang and Yaksh found that the 1000 µg bolus injection of gabapentin caused significant hind limb motor weakness. Generally a bolus injection of 300 µg gabapentin will cause hind limb motor weakness in rats. As 10 µl bolus injections may be used in rats, solutions of gabapentin at a concentration greater than about 30 mg/ml have been of little practical use.

Generally, solutions of gabapentin used in preclinical animal trials contain 0.9% saline in an attempt to approximate physiological saline. However, gabapentin is zwitterionic and may contribute significantly to tonicity of a solution, depending on the concentration of gabapentin. Thus, solutions having high gabapentin concentrations have high tonicity. The presence of 0.9% saline in solutions having a high concentration of gabapentin increases the tonicity, thereby making the solutions hypertonic relative to physiological fluids such as cerebrospinal fluid. For example, the 100 mg/ml gabapentin solution in 0.9% saline described by Wang and Yaksk has a tonicity of about 925 mOsm, as compared to a tonicity of about 300 mOsm for physiological fluids. Hypertonic solutions, when administered to a subject, can result in tissue damage due to shrinkage of cells. In the cerebrospinal fluid, which is generally a poorly mixed tissue compartment, local damage and shrinkage due to hypertonic solutions is a more pressing concern. When administered as a small-volume bolus and flushed with saline or barbotaged with CSF, the risks associated with the administration of a hypertonic fluid are minimal. In contract continuous infusion of a low volume of fluid into the subarachnoid space can result in prolonged exposure of the spinal tissues adjacent to the catheter tip. In this case, the risks associated with administration of a nonphysiological hypertonic solution are increased.

### SUMMARY OF THE INVENTION

An embodiment of the invention provides an injectable solution comprising gabapentin and a solvent. Gabapentin is present in the solution at a concentration greater than about 30 mg/mL, and the solution has a tonicity of less than about 900 mOsm.

In an embodiment, the invention provides an injectable solution of gabapentin, where the solution comprises less than 0.9% (w/v) sodium chloride. In an embodiment, gabapentin may be present in the solution at a concentration greater than about 30 mg/ml.

An embodiment of the invention provides a process for preparing an injectable gabapentin composition. The process comprises mixing gabapentin in a diluent to form a fluid composition, determining the tonicity of the fluid composition, and adding a tonicity enhancing agent to the fluid composition if the tonicity of the fluid composition is determined to be less than between about 290 mOsm to about 320 mOsm. In an embodiment, no tonicity enhancing agent is added to adjust the tonicity of the fluid composition if the tonicity is determined to be greater than about 290 mOsm to about 320 mOsm.

Various embodiments of the invention provide several advantages. For example, solutions having reduced hypertonicity may result in reduced tissue and cell damage due to injection of the solution. By reducing solvent tonicity, increased concentrations of gabapentin may be placed in injectable solutions without rendering the solutions excessively hypertonic. When used in a pump system designed to deliver a therapeutic agent, compositions having increased concentrations of gabapentin will allow for greater time to elapse, relative to compositions having lower gabapentin concentrations, before the pump requires refilling. Increasing time between refills is particularly important when the pump is an implantable pump.

These and other advantages of the invention will become evident upon reading the description herein.

### BRIEF DESCRIPTION OF THE DRAWINGS

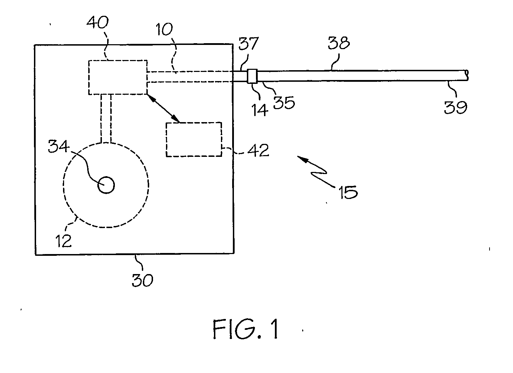

Figure 1 is a diagrammatic illustration of a pump system for delivering a composition comprising a therapeutic agent according to an embodiment of the present invention.

The drawing is not necessarily to scale.

### DETAILED DESCRIPTION

The following description illustrates various embodiments of the invention. It is to be understood that other embodiments of the present invention are contemplated and may be made without departing from the scope or spirit of the present invention. Thus, the following description is not to be taken in a limiting sense.

All scientific and technical terms used in this application have meanings commonly used in the art unless otherwise specified. The definitions provided herein are to facilitate understanding of certain terms used frequently herein and are not meant to limit the scope of the present disclosure.

Embodiments of the present invention provide injectable compositions comprising gabapentin. Injectable compositions comprising gabapentin according to embodiments of the invention may be used for any purpose for which study or use of gabapentin is desired. For example, injectable compositions comprising gabapentin may be used in studies to determine or elucidate (a) the effect of gabapentin on a molecule, cell, tissue, organ, organism, or combination thereof; (b) the mechanism of action of gabapentin, (c) the properties of gabapentin, a solution comprising gabapentin, or a combination thereof; and (d) the like. Injectable compositions comprising gabapentin may also be used as therapy to treat a disease state responsive to gabapentin such as epilepsy, pain, tinnitus, drug addiction, bipolar disorder, osteoarthritis, migraine, and anxiety disorders including social phobia. In the context of the present invention, the terms "treat", "therapy", and the like are meant to include methods to alleviate, slow the progression, prevent, attenuate, or cure the treated disease.

### Injectable Composition

An embodiment of the invention provides an injectable composition comprising gabapentin. As used herein, gabapentin refers to 1-(aminomethyl)cyclohexane acetic acid and pharmaceutically acceptable salts, solvates, hydrates, and polymorphs thereof. 1-(aminomethyl)cyclohexane acetic acid is a γ-aminobutyric acid (GABA) analogue with a molecular formula of C₉H₁₇NO₂ and a molecular weight of 171.24. 1-(aminomethyl)cyclohexane acetic acid is freely soluble in water and in both basic and acidic aqueous solutions. 1-(aminomethyl)cyclohexane acetic acid has a structure of:

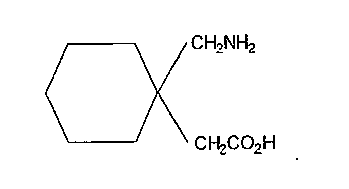

Gabapentin may be obtained from a variety of commercial sources, such as Shanghai Zhongxi International Trading Co., Shanghai, China; Hikal Limited, Bangalore, Karnaraka, India; Erregierre S.p.A., San Paolo d'Argon (BG), Italy; MediChem, SA, Sant Joan Despi (Barcelona), Spain; Ranbaxy Laboratories, New Delhi, India; Procos S.p.A., Cameri, Italy; Zambon Group, Milan, Italy; Hangzhuo Chiral Medicine Chemicals Co., Hangzhuo, China; InterChem Corporation USA, Paramus, NJ; SST Corporation, Clifton, NJ; Teva Pharmaceuticals USA, North Whales, PA; Plantex USA, Hakensack, NJ; and Sigma-Aldrich, St. Louis, MO, or an appropriate distributor. Alternatively, gabapentin may be synthesized and/or prepared as known in the art.

As used herein, "injectable composition" refers to a composition that is fluid at room temperature, which fluid is capable of being injected into a patient. Injectable compositions include solutions, suspensions, dispersions, and the like. Injectable solutions, suspensions, dispersions, and the like may be formulated according to techniques well-known in the art (see, for example, Remington's Pharmaceutical Sciences, Chapter 43, 14th Ed., Mack Publishing Co., Easton, Pa.), using suitable dispersing or wetting and suspending agents, such as sterile oils, including synthetic mono- or diglycerides, and fatty acids, including oleic acid.

Injectable compositions comprising gabapentin may be prepared in water, saline, isotonic saline, phosphate-buffered saline, citrate-buffered saline, and the like and may optionally mixed with a nontoxic surfactant. Dispersions may also be prepared in glycerol, liquid polyethylene, glycols, DNA, vegetable oils, triacetin, and the like and mixtures thereof. Under ordinary conditions of storage and use, these preparations may contain a preservative to prevent the growth of microorganisms. Pharmaceutical dosage forms suitable for injection or infusion include sterile, aqueous solutions or dispersions or sterile powders comprising an active ingredient which powders are adapted for the extemporaneous preparation of sterile injectable or infusible solutions or dispersions. Preferably, the ultimate dosage form is a sterile fluid and stable under the conditions of manufacture and storage. A liquid carrier or vehicle of the solution, suspension or dispersion may be a solvent or liquid dispersion medium comprising, for example, water, ethanol, a polyol such as glycerol, propylene glycol, or liquid polyethylene glycols and the like, vegetable oils, nontoxic glyceryl esters, and suitable mixtures thereof. Proper fluidity of solutions, suspensions or dispersions may be maintained, for example, by the formation of liposomes, by the maintenance of the desired particle size, in the case of dispersion, or by the use of nontoxic surfactants. The prevention of the action of microorganisms can be accomplished by various antibacterial and antifungal agents, for example, parabens, chlorobutanol, phenol, sorbic acid, thimerosal, and the like. In many cases, it will be desirable to include isotonic agents, for example, sugars, buffers, or sodium chloride. Prolonged absorption of the injectable compositions can be brought about by the inclusion in the composition of agents delaying absorption--for example, aluminum monosterate hydrogels and gelatin. Excipients that increase solubility, such as cyclodextrin, may be added.

Sterile injectable compositions may be prepared by incorporating a therapeutic agent in the desired amount in the appropriate solvent with various other ingredients as enumerated above and, as desired, followed by sterilization. Any means for sterilization may be used. For example, the solution may be autoclaved or filter sterilized. In the case of sterile powders for the preparation of sterile injectable solutions, methods of preparation include vacuum drying and freeze-drying techniques, which yield a powder of the active ingredient plus any additional desired ingredient present in a previously sterile-filtered solution.

Injectable compositions comprising gabapentin may be heat treated or sterilized by autoclaving. Because increased temperature may result in increased conversion of gabapentin to its corresponding lactam, which is generally considered more toxic than gabapentin, it would be expected that high temperatures should be avoided when preparing compositions comprising gabapentin. Surprisingly, compositions comprising gabapentin may be sterilized by autoclaving to provide suitable sterile injectable gabapentin compositions. Heat treatment, whether or not through autoclaving, may be performed at any combination of temperature and time necessary to sterilize a composition comprising gabapentin. For example, a composition may be subjected to heat treatment for about 2 minutes to about 60 minutes at temperatures of about 110 °C to about 140 °C. Specific exemplary times and temperatures that may be used include 24 minutes at 121.1 °C, 4 minutes at 130 °C, 30 min at 118 °C, and 6-8 min at 121.1 °C. It will be recognized that with higher temperatures and the longer durations of heat treatment, the likelihood of gabapentin lactam formation will be increased. To prevent excess formation of lactam, the time and temperature of heat treatment may be adjusted to a combination that reduces lactam formation, yet continues to sterilize the composition comprising gabapentin.

In an embodiment, an appropriate weight of non-sterile gabapentin powder is dissolved in an appropriate volume of sterile water for injection to yield an aqueous gabapentin solution. The pH is adjusted to about 6 with 1N NaOH or 1N HCl, and the resulting solution is sterilized by autoclaving.

In an embodiment, an injectable composition comprising gabapentin is an injectable solution comprising an aqueous solvent. The solvent may be water or saline. The saline may be, e.g., 0.9% (w/v) saline or a solution where just enough sodium chloride is added to make the final solution isotonic. The saline may be sterile saline for injection. In an embodiment, the final solution has a pH between about 4 and about 9, between about 5 and about 7, between about 5.5 and about 6.5, or about 6. The pH of an injectable gabapentin composition may be adjusted with a pharmacologically acceptable acid, base, buffer or combination thereof. In an embodiment, pH is adjusted with hydrochloric acid or sodium hydroxide. The hydrochloric acid or sodium hydroxide may be in any suitable form, such as a 1N solution. In an embodiment, an injectable gabapentin solution has a pH in the range of between about 4 and about 9, between about 5 and about 7, or about 6. Preferably, the final solution contains less than about 5% of gabapentin lactam. In an embodiment, the final solution contains less than about 2% gabapentin lactam. In an embodiment, the final solution contains less than about 1% gabapentin lactam.

A composition comprising gabapentin according to an embodiment of the invention includes an amount of gabapentin effective to treat a disease responsive to gabapentin. In an embodiment, the amount of gabapentin is effective to treat a gabapentin-responsive disease when administered intrathecally. Generally, gabapentin may be present in a solution or suspension at a concentration between about 0.1 mg/mL and about 100 mg/mL. In an embodiment, gabapentin is present in a solution or suspension at a concentration between about 10 mg/mL and about 90 mg/mL. In an embodiment, gabapentin is present in a solution or suspension at a concentration between about 20mg/mL and about 80 mg/mL. In an embodiment, gabapentin is present in a solution or suspension at a concentration of about 80 mg/mL.

In an embodiment, an injectable gabapentin composition is substantially free of preservatives, substantially free of buffers, or substantially free of both preservatives and buffers.

### Tonicity

In an embodiment, the invention provides an injectable composition comprising gabapentin, where the composition is substabtially isotonic with a physiological fluid of a subject. For example, the injectable solution may be isotonic with a subject's blood or cerebrospinal fluid. Cerebrospinal fluid typically has a tonicity of about 305 mOsm. Accordingly, an embodiment of the invention provides an injectable gabapentin composition having a tonicity of about 290 mOsm to about 320 mOsm. However, such tonicities are not always achievable with gabapentin compositions. For example, gabapentin dissolved in water at a concentration of 80 mg/ml has a tonicity of about 500 mOsm. When the concentration of gabapentin in an injectable composition renders the composition hypertonic relative to a subject's physiological fluid, it is preferred that little or no amount of a tonicity enhancing agent be added to the composition. As used herein, "tonicity enhancing agent" means a compound or composition that increases tonicity of a composition. However, it will be recognized that it may be desirable to add one or more additional compounds to the composition even though the addition of the additional compound(s) will further increase tonicity of an injectable gabapentin solution. For example, it may be desirable to add to the composition an additional therapeutic agent, stabilizing compound, preservative, solubilizing agent, buffer, etc., even though tonicity will be increased.

An embodiment of the invention provides a process for preparing an injectable gabapentin composition. The method comprises mixing and/or dissolving gabapentin in a diluent or solvent to generate a composition, determining the tonicity of the composition, and adjusting the tonicity of the composition if appropriate. Any diluent or solvent may be used, provided that that the diluent or solvent is pharmacologically acceptable. Preferably gabapentin is stable in the diluent or solvent. In an embodiment, water is used as the diluent. Tonicity may be determined by any means. For example, tonicity may be determined by measuring freezing point depression or decreased vapor pressure (with solutes versus substantially pure solvent). Tonicity may also be estimated. For example, a solution resulting from the mixture of one solution having a tonicity of about 500 mOsm and another solution having a tonicity of about 300 mOsm will have a resulting tonicity between about 300 mOsm and about 500 mOsm. An osmometer may be used to determine tonicity. If the tonicity of the composition is less than between about 290 mOsm to about 320 mOsm, a tonicity enhancing agent may be added to the compostion to increase tonicity to between about 290 mOsm to about 320 mOsm. Any tonicity enhancing agent may be used to increase the tonicity of a composition according to various embodiments of the invention, provided that the tonicity enhancing agent is pharmacologically acceptable. Preferably, a tonicity enhancing agent is compatible with gabapentin. In an embodiment, sodium chloride is used as a tonicity enhancing agent. If the tonicity of a composition comprising gabapentin is greater than about 320 mOsm, a tonicity enhancing agent is preferably not added. In an embodiment, one or more additional agents may be added to the composition prior to determining the tonicity of the composition. For example, an additional therapeutic agent, stabilizing agent, preservative, solubilizing agent, buffer, etc. may be added prior to determining the tonicity of a composition for purposes of determining whether to add a tonicity enhancing agent.

An embodiment of the invention provides an injectable composition comprising gabapentin in a concentration of greater than about 30 mg/ml, where the injectable composition has a tonicity of less than about 900 mOsm. For example, gabapentin may be present in an injectable composition at a concentration of greater than about 31 mg/ml, greater than about 32 mg/ml, greater than about 33 mg/ml, greater than about 34 mg/ml, greater than about 35 mg/ml, greater than about 36 mg/ml, greater than about 37 mg/ml, greater than about 38 mg/ml, greater than about 39 mg/ml, greater than about 40 mg/ml, etc., or between about 30 mg/mL to about 100 mg/mL, between about 30 mg/mL to about 90 mg/mL, between about 40 mg/mL to about 90 mg/mL, or about 80 mg/mL. An injectable gabapentin composition may have a tonicity in the range of, for example, about 250 mOsm to about 700 mOsm, in the range about 250 mOsm to about 600 mOsm, in the range of about 400 mOsm to about 550 mOsm, or about 500 mOsm.

An embodiment of the invention provides an injectable composition comprising gabapentin having a tonicity less than a corresponding composition that is the same as the injectable composition except that the corresponding composition has about 0.9% (w/v) sodium chloride. Thus, the injectable composition may comprise less than about 0.9% (w/v) sodium chloride. The composition may comprise gabapentin in any amount. Preferably, the gabapentin is present in an amount effective to treat a gabapentin-responsive disease when administered to a subject in need thereof. Gabapentin may be present in the injectable composition at a concentration of between about 0.1 mg/ml to about 100 mg/ml, between about 10 mg/ml to about 90 mg/ml, between about 20 mg/ml to about 80 mg/ml, etc. In an embodiment, gabapentin is present in an injectable compostion at a concentration between about 30 mg/mL to about 100 mg/mL, between about 30 mg/mL to about 90 mg/mL, between about 40 mg/mL to about 90 mg/mL, or about 80 mg/mL. In an embodiment, a composition comprises between about 10 mg/ml and about 50 mg/ml gababentin. For example, the composition may comprise between about 20 mg/ml and 40 mg/ml, or about 30 mg/ml.

### Additional Therapeutic Agents

In an embodiment, the invention provides an injectable composition comprising gabapentin and one or more additional therapeutic agents. Any additional therapeutic agent may be included in the injectable composition. Preferably, the additional therapeutic agent is compatible with gabapentin. In an embodiment, gabapentin and at least one of the additional therapeutic agents are useful for treating the same disease state in a subject.

In an embodiment, the invention provides an injectable composition comprising gabapentin and one or more additional therapeutic agents useful for treating tinnitus. Application Serial No. 10/611,459, entitled "A method for treating severe tinnitus", filed July 1, 2003, discusses the use of intrathecally delivered gabapentin for the treatment of tinnitus. In Application Serial No. 10/611,459, local anesthetics, GABA agonists, including GABA_{A} and GABA_{B} agonists, serotonin agonists, thyrotropin-releasing hormones, and benzodiazapines are also discussed as being useful for treating tinnitus. Thus, according to an embodiment, the present invention provides an injectable composition comprising gabapentin and one or more of a local anesthetic, a GABA agonist, a serotonin agonist, a thyrotropin-releasing hormone, and a benzodiazapine. Specific exemplary additional therapeutic agents useful for treating tinnitus include lidocaine, bupivacaine, baclofen, muscimol, sumatriptan, sodium valproate, midazolam, alprazolam, adenosine and pharmacologically acceptable salts thereof. Any useful amount of an additional therapeutic agent may be included in an injectable composition comprising gabapentin. Baclofen, for example, may be present in an injectable composition in a concentration between about 10 and about 4000 mcg/ml, between about 50 and about 2000 mcg/ml, between about 1000 and about 4000 mcg/ml, and between about 20 and about 2000 mcg/ml. It will be recognized that an injectable composition comprising gabapentin and one or more of the above-mentioned additional therapeutic agents may be useful for treating diseases other than tinnitus.

An embodiment of the invention provides an injectable composition comprising gabapentin and one or more additional therapeutic agents useful for treatment of pain. Provisional Application Serial No. 60/513681, entitled "INTRATHECAL GABAPENTIN FOR TREATMENT OF PAIN AND EPILEPSY", filed on October 23, 2003, which provisional application is herein incorporated by reference in its entirety, discusses the use of intrathecally delivered gabapentin for the treatment of pain and epilepsy. In Provisional Application Serial No. 60/513681 analgesics and adjuvant analgesics are discussed as being useful for treating pain. Accordingly, an embodiment of the invention provides an injectable composition comprising gabapentin may further comprise one or more analgesic and/or adjuvant analgesic. Exemplary analgesics include opioids, NSAIDS, local anesthetics, and alpha2-adrenergic agonists. Adjuvent analgesics include anticonvulsants and antidepressants. Specific exemplary analgesics and adjuvant analgesics include, morphine, hydromorphone, bupivacaine, clonidine, baclofen and pharmacologically acceptable salts thereof. Any useful amount of an additional therapeutic agent may be included in an injectable composition comprising gabapentin. For example, morphine sulfate may be present in an injectable composition in a concentration between about 2.5 mg/ml and about 50 mg/ml. Hydromorphone may be present in an injectable composition comprising gabapentin at, for example, a concentration of between about 1 mg/mL and about 20 mg/mL. Baclofen, for example, may be present in an injectable composition in a concentration between about 10 and about 4000 mcg/ml, between about 50 and about 2000 mcg/ml, between about 1000 and about 4000 mcg/ml, and between about 20 and about 2000 mcg/ml. It will be recognized that an injectable composition comprising gabapentin and one or more of the above-mentioned additional therapeutic agents may be useful for treating diseases other than pain.

Injectable compositions comprising gabapentin and an additional therapeutic agent according to an embodiment of the invention may be prepared in any manner that produces a product having pharmacological activity. For example, (a) an injectable composition comprising gabapentin may be mixed with an injectable composition comprising an additional therapeutic agent; (b) solid forms of gabapentin, e.g. gabapentin powder, and an additional therapeutic agent may be mixed, and the resulting mixture may be added to a solvent or diluent to produce an injectable composition; (c) a solid form of gabapentin may be added to an injectable composition comprising an additional therapeutic agent; (d) a solid form of an additional therapeuric agent may be added to an injectable composition comprising gabapentin, (e) etc.

In an embodiment, a sterile injectable solution comprising an additional therapeutic agent and a sterile injectable solution comprising gabapentin are added together. The sterile solutions may be added together in a sterile syringe. Adding together two sterile solutions provides for a convenient and easy means for preparing an injectable composition comprising gabapentin and an additional therapeutic agent, as well as minimizes risks of contamination associated with compounding from a non-sterile powder.

Provided below in Table 1 are examples of how a sterile injectable solution comprising 80 mg/ml gabapentin may be mixed with a sterile injectable solution comprising an opioid agonist. The opioid agonsists listed in Table 1 are commercially available in sterile injectable solutions. INFUMORPH is a preservative-free morphine sulfate sterile solution, and DILAUDID HP is a sterile solution comprising hydromorphone hydrochloride. Both INFUMORPH and DILAUDID HP have a tonicity of about 300 mOsm. In the examples provided in Table 1, the sterile injectable solution comprising 80 mg/ml has a tonicity of about 500 mOsm, although it will be recognized that any injectable gabapentin composition according to various embodiments of the invention may be used. A sterile injectable solution comprising 80 mg/ml gabapentin may be obtained by dissolving an appropriate amount of non-sterile gabapentin powder in water, adjusting the pH to about 6 with 1N NaOH or 1N HCl, and sterilizing the resulting solution. As shown in Table 1, sterile injectable end products may be achieved with reasonable concentrations of gabapentin and opioid. In addition, the tonicity of the resulting end products described in Table 1 are in the range of between about 300 mOsm and about 500 mOsm.

**Table 1: Examples of sterile injectable compositions**

| Common Mixtures Contemplated (V:V) | Infumorph (25 mg/mL) Final Opioid Conc (mg/ml) | Gabapentin (80 mg/mL) Final Gabapentin Conc (mg/ml) |
|---|---|---|
| 50:50 | 12.5 | 40 |
| 90:10 | 22.5 | 8 |
| 75:25 | 18.75 | 20 |
| 10:90 | 2.5 | 72 |
| | Dilaudid HP (10 mg/mL) | Gabapentin(80 mg/mL) |
| 90:10 | 9 | 8 |
| 50:50 | 5 | 40 |
| 10:90 | 1 | 72 |

It will be recognized that an injectable composition comprising gabapentin and little or no additional osmolutes may be serve as a desirable diluent for powder forms of additional therapeutic agents. Because such an injectabele gabapentin composition comprises little or no nonanalgesic osmolutes, more of an additional therapeutic agent may be added to the injectable composition while having minimal impact on the hypertonicity of the final solution. For example, with regard to tonicity, a greater amount of powdered opioid may be added to an injectable solution comprising 80 mg/ml of gabapentin in water than to a solution comprising 80 mg/ml of gabapentin in 0.9% sodium chloride.

### Administration

Injectable compositions according to the invention may be administered to a subject through any acceptable route. For example, the compositions may be administered intravenously, subcutaneously, intra-arterially, inthrathecally, epidurally, intraparenchymally, intraperitoneally, intracerebroventricularly, etc., by infusion or injection.

In an embodiment of the invention, an injectable composition comprising gabapentin is adapted for intrathecal administration. Intrathecal administration of gabapentin provides a means for achieving effective spinal concentrations of gabapentin by bypassing the saturable L-amino acid active transport system and blood-brain barrier, while reducing concomitant systemic or supraspinal drug levels. Any effective amount of gabapentin may be administered intrathecally. For example, gabapentin may be administered intrathecally in a daily dose of between about 0.1 mg and about 200 mg, between about 1 mg and about 150 mg, between about 2 mg and about 60 mg, or greater than about 25 mg. Gabapentin may also be administered in a daily dose of less than about 25 mg. For example, gabapentin may be administered at a daily dose of between about 0.1 mg and about 10 mg, between about 0.1 mg and 5 mg, between about 0.1 mg and 2 mg, between about 0.1 and 1 mg, between about 0.1 and 0.5 mg, or about 0.2 mg. It will be understood that daily dose requirements may be adjusted to account for variability in CSF volume, CSF production rates, and rate of clearance of gabapentin from the CSF. One of skill in the art will understand that such variability may be due in part to, e.g., gender and/or age.

Gabapentin may be administered to a subject using a therapy delivery system 15, as shown in Figure 1. The system 15 comprises a therapy delivery device 30. The device 30 comprises a pump 40 coupled a reservoir 12 for housing an injectable composition comprising gabapentin. The system 15 further comprises a catheter 38. The catheter 38 comprises a proximal portion 35 coupled to the pump and a distal portion 39 adapted for infusing the composition to a directed location of a subject, e.g., a subject's cerebrospinal fluid. It will be recognized that the catheter 38 may have one or more drug delivery regions along the length of the catheter 38 and that a drug delivery region may or may not be at the distal end 39 of the catheter 38. The device 30 may be implantable or may be an external device. The therapy delivery device 30 may have a port 34 into which a hypodermic needle can be inserted to inject a quantity of therapeutic agent into reservoir 12. The device 30 may have a catheter port 37, to which the proximal portion 35 of catheter 38 may be coupled. The catheter port 37 may be coupled to pump 40 through an internal catheter 10. A connector 14 may be used to couple the catheter 38 to the catheter port 37 of the device 30. Device 30 may contain a microprocessor 42 or similar device that can be programmed to control the amount of fluid delivery. Device 30 may take the form of the device shown in U.S. Pat. No. 4,692,147 (Duggan), assigned to Medtronic, Inc., Minneapolis, Minn., commercially available as the Synchromed® infusion pump, which is incorporated by reference. A system 15 comprising (a) a device 30 having a reservoir 12 and (b) a composition comprising gabapentin housed in the reservoir 12 is contemplated by the invention.

### Kit With Instructions

An embodiment of the invention provides a kit comprising an injectable gabapentin composition and instructions indicating that the injectable composition comprising gabapentin may be administered to cerebrospinal fluid of a subject. The instructions may include directions for administering the injectable composition comprising gabapentin to a subject's cerebrospinal fluid through any acceptable route, including for example intrathecally, intracerebroventricularly, etc or combinations thereof. The instructions may further indicate that the injectable gabapentin composition may be placed in an implantable pump 30.

The following patent applications are generally relevant to injectable gabapentin and its use:
US Patent Application Serial No. 10/807,828, entitled INTRATHECAL GABAPENTIN FOR TREATMENT OF PAIN, filed on March 24, 2004;
US Patent Application Serial No. 10/807,827, entitled INTRATHECAL GABAPENTIN FOR TREATMENT OF EPILEPSY, filed on March 24, 2004;
US Patent Application Serial No. 10/508,113, entitled PROCESS FOR PRODUCING INJECTABLE GABAPENTIN COMPOSITIONS, filed on March 24, 2004; and
US Patent Application Serial No. 10/808,054, entitled PUMP SYSTEMS INCLUDING INJECTABLE GABAPENTIN COMPOSITIONS, filed on March 24, 2004.

As those of ordinary skill in the art will readily appreciate upon reading the description herein, at least some of the compositions, devices and methods disclosed in the patents and publications cited herein may be modified advantageously in accordance with the teachings of the present invention.

## Claims

1. A heat-treated, sterile, injectable Pharmaceutical composition comprising gabapentin and a pharmacologically acceptable solvent, wherein the gabapentin is present in the solution at a concentration greater than about 30 mg/mL, and wherein the solution has a tonicity of less than about 900 mOsm.

2. The injectable, composition of claim 1, wherein the composition is an injectable solution.

3. The injectable composition of claim 1 or claim 2, wherein the gabapentin is present in the solution at a concentration in the range of about 30 mg/mL to about 100 mg/mL.

4. The injectable composition of claim 1 or claim 2, wherein the gabapentin is present in the solution at a concentration in the range of about 30 mg/mL to about 90 mg/mL.

5. The injectable composition of claim 1 or claim 2, wherein the gabapentin is present in the solution at a concentration in the range of about 40 mg/mL to about 90 mg/mL.

6. The injectable composition of claim 1 or claim 2, wherein the gabapentin is present in the solution at a concentration of about 80 mg/mL.

7. The injectable composition of any one of claims 2 to 6, wherein the solvent is water.

8. The injectable composition of claim 7 further comprising sodium chloride.

9. The injectable composition of claim 8, wherein the sodium chloride is present in an amount such that the solution is substantially isotonic with cerebrospinal fluid.

10. The injectable composition of any one of claims 1 to 9, wherein the tonicity of the solution is in the range of about 250 mOsm to about 700 mOsm.

11. The injectable composition of any one of claims 1 to 9, wherein the tonicity of the solution is in the range of about 250 mOsm to about 600 mOsm.

12. The injectable composition of any one of claims 1 to 9, wherein the tonicity of the solution is about 500 mOsm.

13. The injectable composition of any one of claims 2 to 12, wherein the solution has a pH between about 4 and about 9.

14. The injectable composition of any one of claims 2 to 12, wherein the solution has a pH between about 5 and about 7.

15. The injectable composition of any one of claims 1 to 14, wherein the solution contains substantially no preservatives.

16. The injectable composition of any one of claims 1 to 15, wherein the solution contains substantially no buffers.

17. The injectable composition of any one of claims 1 to 16, further comprising one or more additional therapeutic agents.

18. The injectable composition of claim 17, wherein at least one of the one or more additional therapeutic agents is selected from the group consisting of: a local anesthetic, a GABA agonist, a serotonin agonist, a thyrotropin-releasing hormone, a benzodiazapine, an opioid agonist, a non-steroidal anti-inflammatory agent, an alpha2-adrenergic agonist, an anticonvulsant agent, and an antidepressant.

19. The injectable composition of claim 17, wherein at least one of the one or more additional therapeutic agents is selected from the group consisting of: morphine, hydromorphone, bupivacaine, clonidine, lidocaine, baclofen, muscimol, sumatriptan, sodium valproate, midazolam, adenosine and alprazolam, or a pharmacologically acceptable salt thereof.

20. The injectable composition of claim 19, wherein at least one of the one or more additional therapeutic agents is morphine or a pharmacologically acceptable salt thereof.

21. The injectable composition of claim 20, wherein the morphine or the pharmacologically acceptable salt thereof is present in the solution at a concentration of about 10 mg/mL to about 50 mg/mL.

22. The injectable composition of claim 19, wherein at least one of the one or more additional therapeutic agents is hydromorphone or a pharmacologically acceptable salt thereof.

23. The injectable composition of claim 22, wherein the hydromorphone is present in the solution at a concentration of about 1 mg/mL to about 20 mg/mL.

24. The injectable composition of claim 6, wherein the pH is between about 5.5 and 6.5, wherein the tonicity is about 500 mOsm, and wherein the solution is substantially free of preservatives and buffers.

25. A process for preparing an injectable gabapentin composition, the process comprising:
mixing gabapentin in a diluent to form a fluid composition;
determining the tonicity of the fluid composition; and
adding a tonicity enhancing agent to the fluid composition if the tonicity of the fluid composition is determined to be less than between about 290 mOsm to about 320 mOsm.

26. The process of claim 25, wherein the tonicity enhancing agent is added to adjust the tonicity of the fluid composition to between about 290 mOsm to about 320 mOsm.

27. The process of claim 25, wherein no tonicity enhancing agent is added to the fluid composition if the tonicity of the fluid composition is determined to be greater than between about 290 mOsm to about 320 mOsm.

28. The process of any one of claims 25 to 27, wherein one or more additional therapeutic agents are added to the fluid composition prior to determining the tonicity of the fluid composition.

29. The process of claim 25, wherein the pH of the fluid composition is adjusted prior to determining the tonicity of the fluid composition.

## Patentansprüche

1. Hitzebehandelte, sterile, injizierbare pharmazeutische Zusammensetzung, die Gabapentin und ein pharmakologisch akzeptables bzw. verträgliches Lösungsmittel enthält, wobei das Gabapentin in der Lösung in einer Konzentration von mehr als etwa 30 mg/ml vorliegt, und wobei die Lösung eine Tonizität von weniger als etwa 900 mOsm aufweist.

2. Injizierbare Zusammensetzung nach Anspruch 1, wobei die Zusammensetzung eine injizierbare Lösung ist.

3. Injizierbare Zusammensetzung nach Anspruch 1 oder 2, wobei das Gabapentin in der Lösung in einer Konzentration im Bereich vom etwa 30 mg/ml bis etwa 100 mg/ml vorliegt.

4. Injizierbare Zusammensetzung nach Anspruch 1 oder 2, wobei das Gabapentin in der Lösung in einer Konzentration im Bereich von etwa 30 mg/ml bis etwa 90 mg/ml vorliegt.

5. Injizierbare Zusammensetzung nach Anspruch 1 oder 2, wobei das Gabapentin in der Lösung in einer Konzentration im Bereich von etwa 40 mg/ml bis etwa 90 mg/ml vorliegt.

6. Injizierbare Zusammensetzung nach Anspruch 1 oder 2, wobei das Gabapentin in der Lösung in einer Konzentration von etwa 80 mg/ml vorliegt.

7. Injizierbare Zusammensetzung nach einem der Ansprüche 2 bis 6, wobei das Lösungsmittel Wasser ist.

8. Injizierbare Zusammensetzung nach Anspruch 7, die ferner Natriumchlorid aufweist.

9. Injizierbare Zusammensetzung nach Anspruch 8, wobei das Natriumchlorid in einer solchen Menge vorliegt, dass die Lösung im Wesentlichen bis Cerebrospinalflüssigkeit isotonisch ist.

10. Injizierbare Zusammensetzung nach einem der Ansprüche 1 bis 9, wobei die Tonizität der Lösung im Bereich von etwa 250 mOsm bis etwa 700 mOsm liegt.

11. Injizierbare Zusammensetzung nach einem der Ansprüche 1 bis 9, wobei die Tonizität der Lösung im Bereich von etwa 250 mOsm bis etwa 600 mOsm liegt.

12. Injizierbare Zusammensetzung nach einem der Ansprüche 1 bis 9, wobei die Tonizität der Lösung etwa 500 mOsm beträgt.

13. Injizierbare Zusammesetzung nach einem der Ansprüche 2 bis 12, wobei die Lösung einen pH-Wert zwischen etwa 4 und etwa 9 aufweist.

14. Injizierbare Zusammensetzung nach einem der Ansprüche 2 bis 12, wobei die Lösung einen pH-Wert zwischen etwa 5 und etwa 7 aufweist.

15. Injizierbare Zusammensetzung nach einem der Ansprüche 1 bis 14, wobei die Lösung im Wesentlichen keine Konservierungsmittel enthält.

16. Injizierbare Zusammensetzung nach einem der Ansprüche 1 bis 15, wobei die Lösung im Wesentlichen keine Puffer enthält.

17. Injizierbare Zusammensetzung nach einem der Ansprüche 1 bis 16, die ferner einen oder mehrere therapeutische Wirkstoffe aufweist.

18. Injizierbare Zusammensetzung nach Anspruch 17, wobei wenigstens einer des einen oder der mehreren zusätzlichen therapeutischen Wirkstoffe aus der Gruppe ausgewählt ist, die besteht aus: einem Lokalanästhetikum, einem GABA-Agonisten, einem Serotonin-Agonisten, einem Thyrotropinfreisetzenden Hormon, einem Benzodiazapin, einem Opioid-Agonisten, einem nicht-steroidalen antiinflammatorischen Wirkstoff, einem alpha2-adrenergischen Agonisten, einem antikonvulsivischen Wirkstoff und einem Antidepressivum.

19. Injizierbare Zusammensetzung nach Anspruch 17, wobei wenigstens einer des einen oder der mehreren zusätzlichen therapeutischen Wirkstoffe aus der Gruppe ausgewählt ist, die besteht aus: Morphin, Hydromorphon, Bupivacain, Clonidin, Lidocain, Baclofen, Muscimol, Sumatriptan, Natrium-Valproat, Midazolam, Adenosin und Alprazolam oder einem pharmakologisch verträglichen Salz hiervon.

20. Injizierbare Zusammensetzung nach Anspruch 19, wobei wenigstens einer des einen oder der mehreren zusätzlichen therapeutischen Wirkstoffe Morphin oder ein pharmakologische verträgliches Salz hiervon ist.

21. Injizierbare Zusammensetzung nach Anspruch 20, wobei das Morphin oder das pharmakologische verträgliche Salz hiervon in der Lösung in einer Konzentration von etwa 20 mg/ml bis etwa 50 mg/ml vorliegt.

22. Injizierbare Zusammensetzung nach Anspruch 19, wobei wenigstens einer des einen oder der mehreren zusätzlichen therapeutischen Wirkstoffe Hydromorphon oder ein pharmakologisch verträgliches Salz hiervon ist.

23. Injizierbare Zusammensetzung nach Anspruch 22, wobei das Hydromorphon in der Lösung in einer Konzentration von etwa 1 mg/ml bis etwa 20 mg/ml vorliegt.

24. Injizierbare Zusammensetzung nach Anspruch 6, wobei der pH-Wert zwischen etwa 5,5 und 6,5 liegt, wobei die Tonizität etwa 500 mOsm beträgt, und wobei die Lösung im Wesentlichen frei von Konservierungsmitteln und Puffern ist.

25. Verfahren zur Herstellung einer injizierbaren Gabapentin-Zusammensetzung, wobei das Verfahren beinhaltet: Mischen von Gabapentin in einem Verdünnungsmittel zur Ausbildung einer Fluidzusammensetzung;
Bestimmen der Tonizität der Fluidzusammensetzung; und
Zugeben eines Tonizitätsverbesserungswirkstoffs zu der Fluidzusammensetzung, wenn bestimmt wird, dass die Tonizität der Fluidzusammensetzung weniger als zwischen etwa 290 mOsm bis etwa 320 mOsm beträgt.

26. Verfahren nach Anspruch 25, wobei der Tonizitätsverbesserungswirkstoff zum Einstellen der Tonizität der Fluidzusammensetzung auf zwischen etwa 290 mOsm bis etwa 320 mOsm zugegeben wird.

27. Verfahren nach Anspruch 25, wobei kein Tonizitätsverbesserungswirkstoff zu der Fluidzusammensetzung zugegeben wird, wenn bestimmt wird, dass die Tonizität der Fluidzusammensetzung mehr als etwa 290 mOsm bis etwa 320 mOsm beträgt.

28. Verfahren nach einem der Ansprüche 25 bis 27, wobei einer oder mehrere zusätzliche therapeutische Wirkstoffe zu der Fluidzusammensetzung vor der Bestimmung der Tonizität der Fluidzusammensetzung zugegeben werden.

29. Verfahren nach Anspruch 25, wobei der pH-Wert der Fluidzusammensetzung vor der Bestimmung der Tonizität der Fluidzusammensetzung bestimmt wird.

## Revendications

1. Composition pharmaceutique injectable, stérile, thermiquement traitée, comportant de la gabapentine et un solvant pharmacologiquement acceptable, dans laquelle la gabapentine est présente dans la solution à une concentration supérieure à environ 30 mg/ml, et dans laquelle la solution a une tonicité inférieure à environ 900 mOsm.

2. Composition injectable selon la revendication 1, la composition étant une solution injectable.

3. Composition injectable selon la revendication 1 ou la revendication 2, dans laquelle la gabapentine est présente dans la solution à une concentration dans la plage d'environ 30 mg/ml à environ 100 mg/ml.

4. Composition injectable selon la revendication 1 ou la revendication 2, dans laquelle la gabapentine est présente dans la solution à une concentration dans la plage d'environ 30 mg/ml à environ 90 mg/ml.

5. Composition injectable selon la revendication 1 ou la revendication 2, dans laquelle la gabapentine est présente dans la solution à une concentration dans la plage d'environ 40 mg/ml à environ 90 mg/ml.

6. Composition injectable selon la revendication 1 ou la revendication 2, dans laquelle la gabapentine est présente dans la solution à une concentration d'environ 80 mg/ml.

7. Composition injectable selon l'une quelconque des revendications 2 à 6, dans laquelle le solvant est de l'eau.

8. Composition injectable selon la revendication 7, comportant en outre du chlorure de sodium.

9. Composition injectable selon la revendication 8, dans laquelle le chlorure de sodium est présent en une quantité telle que la solution est sensiblement isotonique avec du fluide cérébrospinal.

10. Composition injectable selon l'une quelconque des revendications 1 à 9, dans laquelle la tonicité de la solution est dans la plage d'environ 250 mOsm à environ 700 mOsm.

11. Composition injectable selon l'une quelconque des revendications 1 à 9, dans laquelle la tonicité de la solution est dans la plage d'environ 250 mOsm à environ 600 mOsm.

12. Composition injectable selon l'une quelconque des revendications 1 à 9, dans laquelle la tonicité de la solution est d'environ 500 mOsm.

13. Composition injectable selon l'une quelconque des revendications 2 à 12, dans laquelle la solution a un pH entre environ 4 et environ 9.

14. Composition injectable selon l'une quelconque des revendications 2 à 12, dans laquelle la solution a un pH entre environ 5 et environ 7.

15. Composition injectable selon l'une quelconque des revendications 1 à 14, dans laquelle la solution ne contient pratiquement aucun agent de conservation.

16. Composition injectable selon l'une quelconque des revendications 1 à 15, dans laquelle la solution ne contient pratiquement aucun tampon.

17. Composition injectable selon l'une quelconque des revendications 1 à 16, comportant en outre un ou plusieurs agents thérapeutiques supplémentaires.

18. Composition injectable selon la revendication 17, dans laquelle au moins un parmi l'agent ou les agents thérapeutiques supplémentaires est choisi parmi le groupe constitué de : un anesthésique local, un agoniste GABA, un agoniste de la sérotonine, une hormone de libération de la thyrotropine, une benzodiazapine, un agoniste opioïde, un agent anti-inflammatoire non stéroïdien, un agoniste alpha2adrénergique, un agent anticonvulsif et un antidépresseur.

19. Composition injectable selon la revendication 17, dans laquelle au moins un parmi l'agent ou les agents thérapeutiques supplémentaires est choisi parmi le groupe constitué de : morphine, hydromorphone, bupivacaïne, clonidine, lidocaïne, baclofène, muscimol, sumatriptan, valproate de sodium, midazolam, adénosine et alprazolam, ou un sel pharmacologiquement acceptable de ceux-ci.

20. Composition injectable selon la revendication 19, dans laquelle au moins un parmi l'agent ou les agents thérapeutiques supplémentaires est la morphine ou un sel pharmacologiquement acceptable de celle-ci.

21. Composition injectable selon la revendication 20, dans laquelle la morphine ou le sel pharmacologiquement acceptable de celle-ci est présent dans la solution à une concentration d'environ 10 mg/ml à environ 50 mg/ml.

22. Composition injectable selon la revendication 19, dans laquelle au moins un parmi l'agent ou les agents thérapeutiques supplémentaires est l'hydromorphone ou un sel pharmacologiquement acceptable de celle-ci.

23. Composition injectable selon la revendication 22, dans laquelle l'hydromorphone est présente dans la solution à une concentration d'environ 1 mg/ml à environ 20 mg/ml.

24. Composition injectable selon la revendication 6, dans laquelle le pH est compris entre environ 5,5 et 6,5, dans laquelle la tonicité est d'environ 500 mOsm, et dans laquelle la solution est pratiquement exempte d'agents de conservation et de tampons.

25. Procédé pour préparer une composition de gabapentine injectable, le procédé comportant les étapes consistant à : mélanger de la gabapentine dans un diluant pour former une composition fluide ;
déterminer la tonicité de la composition fluide ; et
ajouter un agent améliorateur ou renforçateur de tonicité à la composition fluide s'il est déterminé que la tonicité de la composition fluide est inférieure à une valeur entre environ 290 mOsm et environ 320 mOsm.

26. Procédé selon la revendication 25, dans lequel l'agent renforçateur de tonicité est ajouté pour ajuster la tonicité de la composition fluide jusqu'à entre environ 290 mOsm et environ 320 mOsm.

27. Procédé selon la revendication 25, dans lequel aucun agent renforçateur de tonicité n'est ajouté à la composition fluide s'il est déterminé que la tonicité de la composition fluide est supérieure à une valeur entre environ 290 mOsm et environ 320 mOsm.

28. Procédé selon l'une quelconque des revendications 25 à 27, dans lequel un ou plusieurs agents thérapeutiques supplémentaires sont ajoutés à la composition fluide avant de déterminer la tonicité de la composition fluide.

29. Procédé selon la revendication 25, dans lequel le pH de la composition fluide est ajusté avant de déterminer la tonicité de la composition fluide.
